# NEUE EUROPÄISCHE PATENTSCHRIFT

(11) **EP 0 613 499 B2**
(45) Veröffentlichungstag und Bekanntmachung des Hinweises auf die Entscheidung über den Einspruch: **30.03.2005**
(45) Hinweis auf die Patenterteilung: 11.02.1998
(21) Anmeldenummer: 92923502.6
(22) Anmeldetag: 13.11.1992
(51) Int. Cl.: C12N 15/24, A61K 38/20, C07K 14/54, C12P 21/02

(54) **MENSCHLICHE IL-4 MUTANTENPROTEINE**
HUMAN IL-4 MUTANT PROTEINS
PROTEINES MUTANTES DE L'IL-4 HUMAINE MUTANTE

(30) Priorität: 13.11.1991 DE 4137333
(43) Veröffentlichungstag der Anmeldung: 07.09.1994
(73) Patentinhaber: BAYER AG, 51368 Leverkusen (DE)
(72) Erfinder: SEBALD, Walter, Prof. Dr., D-97074 Würzburg (DE)
(86) Internationale Anmeldenummer: PCT/EP1992/002614
(87) Internationale Veröffentlichungsnummer: WO 1993/010235

(56) Entgegenhaltungen:
- FEBS LETTERS, Bd. 286, Nr. 1, 2 Juli 1991, AMSTERDAM NL, Seiten 58-60; NIELS KRUSE ET AL: 'Site-directed mutagenesis reveals the importance of disulfide bridges and aromatic residues for structure and proliferative activity of human Interleukin-4'
- EMBO JOURNAL, Bd. 11, Nr. 9, September 1992, EYNSHAM, OXFORD GB, Seiten 3237-3244; N. KRUSE ET AL: 'Conversion of human interleukin-4 into a high affinity antagonist by a single amino acid replacement'
- EUROPEAN JOURNAL OF BIOCHEMISTRY, Bd. 180, 1989, BERLIN, Seiten 295-300; U. WEIGEL ET AL: 'Mutant proteins of human interleukin 2 . Renaturation yield,proliferative activity and receptor binding' in der Anmeldung erwähnt
- BIOLOGICAL CHEMISTRY HOPPE-SEYLER, Bd. 373, Nr. 9, September 1992, Seiten 789-790; N. KRUSE ET AL: 'Mutational analysis of human Interleukin-4 : Identification of crucial amino acids for receptor binding and generation of a high affinity antagonist'

## Beschreibung

Breite Bevölkerungsschichten leiden heute an Allergien. Die zunehmende Luftverschmutzung und die zunehmende Zahl an diffus in der Umwelt vorhandenen, allergieauslösenden Substanzen läßt erwarten, daß die Zahl der Erkrankungen, insbesondere bei Kindern, in Zukunft noch weiter steigen wird. Deshalb ist es dringend notwendig, Medikamente zu entwickeln, die in den Ablauf allergischer Prozesse eingreifen können.

Humanes Interleukin-4 (hIL-4) ist eines unter den zahlreichen Cytokinen, die die Proliferation, die Reifung, das überleben und die Differenzierung lymphoider und myeloischer Zellen induzieren und koodinieren (1-3). Insbesondere ist hIL-4 an der IgE-medi-ierten Immunreaktion beteiligt und beschleunigt direkt die Proliferation von Thymocyten und aktivierten T-Zellen. Man konnte ein hochaffines IL-4-Rezeptorprotein mit Mr 140 000 identifizieren, welches gemäß seiner cDNA-Sequenz aus 800 Aminosäureresten besteht (4). Dieses gehört zu einer kürzlich beschriebenen Gruppe von Rezeptoren, die man als Hämatopoietin-Rezeptor-Superfamilie bezeichnet (5).

Die Aminosäuresequenz des reifen IL-4 besteht aus 129 Resten, wenn man die klonierte cDNA (6) zugrundelegt. Die cDNA ist in E. coli (7, 8) und Hefe (9) exprimiert worden. Aus diesen Quellen kann rekombinantes IL-4 mit hoher biologischer Aktivität gewonnen werden.

In (13) ist ein hIL-4-Mutantenprotein beschrieben, welches einen Austausch in der Position 124 hat. Dieses Mutantenprotein wird als biologisch inaktiv bezeichnet.

Die Rolle des Interleukin 4 bei allergischen Prozessen läßt hoffen, daß Substanzen, die Interteukin-4-vermittelte Prozesse inhibieren oder mit dem hIL-4 konkurrieren, die krankheitsauslösende Reaktionskette unterbrechen.

Es ist deshalb Aufgabe der Erfindung, therapeutische Mittel zur Verfügung zu stellen, deren aktive Bestandteile Antagonisten oder partielle Agonisten des menschlichen Interleukins 4 sind.

In jüngster Zeit ist bereits ein monoclonaler Antikörper bekannt geworden, der gegenüber dem menschlichen Interleukin-4 antagonistische Eigenschaften aufweist (10). Dieser Antikörper enthält ein Fab-Fragment und wird von einer Mensch-Mensch-Hybridomazelllinie produziert. Auch eine Hybridomazellinie aus Milzzellen einer gegen (nicht-)glycosyliertes menschliches IL-4 immunisierten Ratte produziert monoclonale Antikörpergen hIL-4 (11).

Die gestellte Aufgabe wurde vorliegend gelöst durch die Verwendung von hIL-4 Mutantenproteinen, die Antagonisten oder partielle Agonisten des hIL-4 sind, worin an einer oder mehreren der Positionen 121, 124 oder 125. die dort natürlicherweise im Wildtyp auftretende(n) Aminosäure(n) gegen eine bzw. mehrere andere der möglichen natürlichen Aminosäuren ausgetauscht ist, zur Herstellung von Arzneimitteln.

Die erfindungsgemäß verwendeten Muteine haben den Vorteil, daß sie durch ihre Ähnlichkeit mit dem Wildtyp hIL-4 mit diesem in Konkurrenz bei der Besetzung des hll-4-Rezeptors treten.

Es ist weiterhin Aufgabe der Erfindung, hIL-4-Mutantenproteine sowie Verfahren zur deren Herstellung bereitzustellen.

hIL-4 läßt sich als rekombinates Protein (rhIL-4) gentechnologisch, z. B. in E. coli, erzeugen. Das dabei gebildete Protein läßt sich solubilisieren, renaturieren und isolieren. Das rHIL-4 besitzt dann eine hohe spezifische biologische Aktivität, die z. B. durch Messung der DNA-Synthese/Proliferation von aktivierten T-Zellen oder der CD23 Expression von aktivierten B-Zellen bestimmt werden kann (siehe z. B. Kruse, N. et al. (1991) FEBS Lett. 286. 58-60; Kikutani, H. et al. (1986) Cell 47 657-665).

Erfindungsgemäß wurde nun ein Verfahren konzipiert, mit dem sich Mutantenproteine des hIL-4-Wildtyps produzieren lassen, welche die Eigenschaften von hIL-4 Antagonisten oder partiellen hIL-4 Agonisten besitzen. Besonders die Antagonisten des hIL-4 bieten die Möglichkeit, spezifisch die Wirkung des hIL-4 zu hemmen. Dazu wird
- cDNA, die einen DNA-Bereich umfaßt, der den maturen Bereich von hIL-4 kodiert, einer gezielten Oligonucleotid-Mutagenese (site-directed mutagenesis) derart unterworfen, daß an der oder den gewirnschten Position(en) eine ausgewählte andere der möglichen natürlichen Aminosäuren exprimiert wird, oder durch ein Stop-Kodon ein Abbruch der Polypeptidkette erzeugt wird,
- der DNA-Bereich, der den mutierten maturen Bereich von hIL-4 kodiert, in einen Expressionsvektor integriert,
- der gebildete Hybrid-Vektor in E. coli eingesetzt und
- das hIL-4-Mutantenprotein exprimiert und gegebenenfalls isoliert.

Zur Beschaffung von cDNA, die einen DNA-Bereich umfaßt, der den maturen Bereich von hIL-4 kodiert, oder die den maturen Bereich von hll-4 kodiert, wird auf (6) und die dort angeführte Literatur verwiesen. Im vorliegenden Zusammenhang werden unter "cDNA, die den maturen Bereich von hIL-4 kodiert" auch cDNAs verstanden, die bei etwa gleicher Anzahl von Basenpaaren Mutanten der konkret im genannten Stand der Technik angegebenen cDNA darstellen, sofern die damit vorzusehenden hIL-4-Muteine ebenfalls Antagonisten oder partielle Agonisten sind.

Hinsichtlich der Durchnumerierung des den maturen Bereich von hIL-4 kodierenden DNA-Bereichs wird hier Garr, C. et al. (Biochemistry (1991) 30, 1515-1523) gefolgt.

cDNA, die den maturen Bereich von hIL-4 kodiert, kann durch das Ausschneiden eines EcoRV/BamHI-Fragment aus einer gentechnologisch hergestellten cD-NA (z. B. von Britisch Bio-Technology Ltd., Oxford, England) gewonnen werden. Das DNA-Fragment wird unter Zusatz von synthetischen Oligonucleotiden, z.B. 5'-CATGCACAAGTGCGAT und 5'-ATCGCACTTGTG, welche die ersten 4 Aminosäure-Kodons von Interleukin-4 und zusätzlich das Kodon für das Start-Methionin enthalten, zwischen einen Expressionsvektor integriert, beispielsweise zwischen die Ncol- und BamHI-Schnittstellen des Expressionsvektors R^{TS} pRC 109 (12).

Die site-directed-mutagenesis kann gemäß Kramer et al. durchgeführt werden (Nucleic Acids Research (1984) 12, 9441-9455; Cell (1984) 38, 879-887; und Boehringer-Mannheim-Prospekt, Biochemicals for Molecular Biology (1987) 35 etc., siehe auch (12). Das zur Mutagenese verwendete Oligonucleotid kann etwa 6 bis 10 Basen vor und etwa 6 bis 10 Basen hinter der (den) zu ändernden Base(n) enthalten.

Der Fachmann ist auch mit dem Herausschneiden des den mutierten maturen Bereich von hIL-4 kodierenden DNA-Bereichs aus dem Vektor, dem überführen in einen Expressionsvektor, dem Einsetzen in E. coli sowie dem Exprimieren des hIL-4-Mutantenproteins und ihrer fakultativen Isolierung vertraut (McCarthy et al.; Gene (1986) 41, 201-206; Kato et al.; Biochem. Biophys. Res. Commun (1985) 130, 692-699), wobei Modifikationen (12) möglich sind.

Gemäß einer speziellen Ausführungsform des erfindungsgemäßen Verfahrens baut man in die cDNA, die den DNA-Bereich umfaßt, der den maturen Bereich von hIL-4 kodiert, DNA für ein Initiator-Methionin ein, bevor man die gezielte Oligonucleotidmutagenese durchführt.

Gemäß einer weiteren speziellen Ausführungsform kann man den DNA-Bereich, der den mutierten maturen Bereich von hIL-4 kodiert, aus der cDNA-Mutante als Ncol/BamHI-Fragment herausschneiden.

Vorzugsweise verwendet man für die Expression einen temperatur-regulierten Expressionsvektor, beispielsweise piLA502 (ohne linksständigen lambda-Promotor und Polylinker) und/oder einen gängigen E. coli-Stamm als Host, beispielsweise JM103 (recA-). Für pILA502 vergleiche man Schauder et al. (Gene (1987) 52, 279-283). Weitere geeignete Expressionsverfahren lassen sich von Pharmacia beziehen ("Prokaryotic Gene Fusion Vektors"). Der E. coli Stamm JM103 läßt sich gleichfalls von Pharmacia beziehen.

Nachstehend wird die Erfindung durch zwei Beispiele näher erläutert.

### Beispiel 1

Es wurde cDNA, die den maturen Bereich von hiL-4 und ein Initiator-Methionin kodierte, einer gezielten Oligonucleotid-Mutagenese gemäß Kramer et al. unterworfen. Das synthetische Oligonucleotid umfaßte 6 Basen vor und nach dem auszutauschenden Nucleotid und war mit Hilfe einer DNA-Synthese-Maschine hergestellt worden (Applied Biosystems, Modell 380). Die auszutauschende Stelle war Position 124 im C-terminalen, wahrscheinlich α-helicalen Bereich (Positionen 110 bis 129). Dabei wurde Tyr gegen Gly ausgetauscht. Die erhaltene Mutation wurde durch DNA-Sequenzanalyse einzelsträngiger Bakteriophagen-DNA verifiziert. Die mutierte cDNA wurde als Ncol/BamHI-Fragmant aus der doppelsträngigen viralen DNA herausgeschnitten und mit einem temperaturregulierten Expressionsvektor kombiniert, der pILA502 mit der Ausnahme entsprach, daß der linksständige lambda-Promotor und der Polylinker fehlten. Als Host wurde ein recA- Derivat des E. coli-Stammes JM103 verwendet. Die integrierte hIL-4-cDNA wurde sequenziert, um die Mutation zu bestätigen. Danach wurde der Stamm für die Expression des Muteins eingesetzt.

Nach Expression und Isolierung zeigte sich, daß das Mutein (Y124G) mit unveränderter Affinität an den Rezeptor für hIL-4 bindet. Die maximal induzierbare Proliferation von aktivierten peripheren T-Zellen beträgt jedoch nur 10 - 20 % der durch hIL-4-Wildtyp induzierbaren Proliferation. Dies zeigt, daß Mutein Y124G die Eigenschaften eines partiellen Agonisten besitzt.

### Beispiel 2

Es wurde Beispiel 1 mit der Ausnahme wiederholt, daß bei der Position 124 Asp statt Tyr exprimiert wurde. Das isolierte Mutein Y124D zeigt sogar bei einer 1 µM Konzentration keine Aktivität gegen aktivierte periphere T-Zellen. Es hemmt jedoch die Aktivität des hIL-4-Wildtyps mit einer Hemmkonstante von etwa 600 pM. Dies zeigt, daß Mutein Y124D die Eigenschaften eines Antagonisten hat. Mutein Y124D hat eine kleine Restaktivität bei der Induktion von CD23 auf aktivierte B-Zellen. Die maximal erreichbare Induktion beträgt jedoch nur ca. 5 % der durch hIL-4-Wildtyp erreichbaren Induktion. Die Aktivität des hIL-4-Wildtyp wird in diesem System durch Mutein Y124D mit einer Hemmkonstante Kᵢ von etwa 800 pM gehemmt. Mutein Y124D hat in dem B-Zell-System also die Eigenschaften eines sehr schwachen Agonisten.

### Beispiel 3

Es wurde Beispiel 1 mit der Ausnahme wiederholt, daß bei der Position 121 Asp statt Arg exprimiert wurde. Das isolierte Mutein zeigte eine unveränderte Bindung an den hIL-4 Rezeptor. Die maximal induzierbare Proliferation von aktivierten peripheren T-Zellen betrug jedoch nur etwa 30 % der durch hIL-4-Wildtyp induzierbaren Proliferation. Dies zeigt, daß Mutein R 121 D die Eigenschaften eines partiellen Agonisten besitzt.

### Beispiel 4

Es wurde Beispiel 1 mit der Ausnahme wiederholte, daß bei der Position 125 Asp statt Ser exprimiert wurde. Das isolierte Mutein S 125 D band mit unveränderter Affinität an den Rezeptor für hIL-4. Die maximal induzierbare Proliferation von aktivierten peripheren T-Zellen beträgt jedoch nur etwa 35 % der durch hIL-4-Wildtyp induzierbaren Proliferation.

### Literaturverzeichnis:

1) Arai, K.I., Lee, F., Miyajima, A., Miyatake, S., Arai, N. and Yokota, T. (1990) Annu. Rev. Biochem. 59, 783-836.
2) Finkelman, F.D., Holmes, J., Katona, I.M., Urban, J.F., Beckmann, M.P., Park, LS., Schooley, K.A., Coffman, R.L., Mosmann, T.R. and Paul, W.E. (1990) Annu. Rev. Immunol. 8, 303-333.
3) Yokota, T., Arai, N., De Vries, J., Spits, H., Banchereau, J., Zlotnik, A., Rennick, D., Howard, M., Takebe, Y., Miyatake, S., Lee, F. and Arai, K.I. (1988) Immunol. Rev. 102, 137-187.
4) Idzerda, R.L., March, C.J., Mosley, B., Lyman, S. D., Vanden Bos, T., Gimpel, S.D., Din, W.S., Grabstein, K.H., Widmer, M.B., Park, L.S., Cosman, D. and Beckmann, M.P. (1990) J. Exp. Med. 171, 861-873.
5) Cosman, D., Lyman, S.D., Idzerda, R.L., Beckmann, M.P., Park, L.S., Goodwin, R.G. and March, C.J. (1990) Trends Biochem. Sci. 15, 265-270.
6) Yokota, T., Otsuka, T., Mosmann, T., Banchereau, J., DeFrance, T., Blanchard, D., De Vries, J. E., Lee, F. and Arai, K.I. (1986) Proc. Natl. Acad. Sci. USA 83, 5894-5898.
7) Van Kimmenade, A., Bond., M.W., Schumacher, J.H., Laquoi, C. and Kastelein, R.A. (1988) Eur. J. Biochem. 173, 109-114.
8) Jayaram, B. Bevos, R., Guisez, Y. and Fiers, W. (1989) Gene 79, 345-354.
9) Solari, R., Quint, D., Obray, H. McNamee, A., Bolton, E., Hissey, P., Champion, B., Zanders, E., Chaplin, A., Coomber, B., Watson, M., Roberts, B. and Weir, M. (1989) Biochem. J. 262, 897-908.
10) Coffman, R.L.; de Vries, J.E. (Schering Biotech Corp., USA), EP 327283 A1.
11) Abrams, J.S.; Chretien, I.; Lee, F.D.; Pearce, M. K. (Schering Biotech Corp., USA) EP 314402 A2.
12) Weigel, U., Meyer, M. und Sebald, W. (1989) Eur. J. Biochem. 180, 295-300.
13) Niels Kruse et al. (1991) FEBS LETTERS, Bd. 286, Nr. 12, Seiten 58 bis 60.

## Patentansprüche

1. Verwendung von hIL-4 Mutantenproteinen, die Antagonisten oder partielle Agonisten des hIL-4-sind, worin an einer oder mehreren der Positionen 121, 124 oder 125 die dort natürlicherweise im Wildtyp auftretende(n) Aminosäure(n) gegen eine bzw. mehrere andere der möglichen natürlichen Aminosäuren ausgetauscht ist, zur Herstellung von Arzneimitteln.

2. Verwendung nach Anspruch 1, **dadurch gekennzeichnet, dass** in den hlL-4 Mutantenproteinen an Position 124 die dort natürlicherweise auftretende Aminosäure Tyrosin gegen eine andere der möglichen natürlichen Aminosäuren ausgetauscht ist.

3. Verwendung nach Anspruch 2, **dadurch gekennzeichnet, dass** an Position 124 Tyrosin gegen Asparaginsäure oder gegen Glycin ausgetauscht ist.

4. Verwendung nach Anspruch 1, **dadurch gekennzeichnet, dass** in den hIL-4 Mutantenproteinen an Position 121 die dort natürlich auftretende Aminosäure Arginin gegen eine andere der möglichen natürlichen Aminosäuren ausgetauscht ist.

5. Verwendung nach Anspruch 4, **dadurch gekennzeichnet, dass** an Position 121 Arginin gegen Asparginsäure oder gegen Glycin ausgetauscht ist.

6. hIL-4 Mutantenproteine, **dadurch gekennzeichnet, dass** an einer oder mehreren Positionen 121 oder 125 und fakultativ zusätzlich an Position 124 die dort natürlicherweise auftretende(n) Aminosäure(n) gegen eine andere der möglichen natürlichen Aminosäuren ausgetauscht ist.

7. hIL-4 Mutantenproteine, **dadurch gekennzeichnet, dass** an Position 124 die dort natürlicherweise auftretende Aminosäure Tyrosin gegen eine andere der möglichen natürlichen Aminosäuren, mit Ausnahme von Asparaginsäure, ausgetauscht ist.

8. hIL-4 Mutantenproteine nach Anspruch 7, **dadurch gekennzeichnet, dass** Tyrosin gegen Glycin ausgetauscht ist.

9. Verfahren zum Herstellen von hIL-4 Mutantenproteinen nach einem der Ansprüche 6 bis 8, **dadurch gekennzeichnet, dass** man
- cDNA, die einen DNA Bereich umfasst, der den maturen Bereich von hIL-4 kodiert, einer gezielten Oligonucleotid-Mutagenese (site-directed-mutagenisis) derart unterwirft, dass an der oder den gewünschten Position(en) eine ausgewählte andere der möglichen natürlichen Aminosäuren exprimiert wird,
- den DNA-Bereich, der den mutierten maturen Bereich von hlL-4-kodiert, in einen Expressionsvektor einsetzt,
- den gebildeten Hybrid-Vektor in E. coli, Hefe oder einer anderen Eucaryontenzelle einführt und
- die hIL-4 Mutantenproteine exprimiert und gegebenenfalls isoliert.

10. Verfahren nach Anspruch 9, **dadurch gekennzeichnet, dass** man in die cDNA, die den DNA-Bereich umfasst, der den maturen Bereich von hIL-4 kodiert, DNA für ein Initiator-Methionin einbaut, bevor man die gezielte Oligonucleotid-Mutagenese durchführt.

11. Verfahren nach Anspruch 9 oder 10, **dadurch gekennzeichnet, dass** man den DNA-Bereich, der den mutierten maturen Bereich von hlL-4 kodiert, aus dem Vektor als Ncol/BamHI-Fragment herausschneidet.

12. Verfahren nach einem der Ansprüche 9 bis 11, **dadurch gekennzeichnet, dass** man einen temperaturregulierten Expressionsvektor, beispielsweise pILA 502 (ohne linksständigen lambda-Promoter und Polylinker) und/oder den modifizierten E. coli Stamm JM103 (recA) als Host verwendet.

## Claims

1. Use of mutant hIL-4 proteins which are antagonists or partial agonists of hIL-4, and in which one or more of the amino acids which occur naturally in the wild type at positions 121, 124 or 125 is/are, respectively, replaced by (a) different amino acid(s) of the possible natural amino acids, for preparing medicaments.

2. Use according to Claim 1, **characterized in that**, in the mutant hIL-4 proteins, the amino acid tyrosine which occurs naturally at position 124 is replaced by a different amino acid of the possible natural amino acids.

3. Use according to Claim 2, **characterized in that**, at position 124, tyrosine is replaced by aspartic acid or by glycine.

4. Use according to Claim 1, **characterized in that**, in the mutant hIL-4 proteins, the amino acid arginine which occurs naturally at position 121 is replaced by a different amino acid of the possible natural amino acids.

5. Use according to Claim 4, **characterized in that**, at position 121, arginine is replaced by aspartic acid or by glycine.

6. Mutant hIL-4 proteins, **characterized in that** one or more of the amino acids which occur(s) naturally at positions 121 or 125, and also facultatively at position 124, is/are, respectively, replaced by a different amino acid of the possible natural amino acids.

7. Mutant hIL-4 proteins, **characterized in that** the amino acid tyrosine which occurs naturally at position 124 is replaced by a different amino acid of the possible natural amino acids, with the exception of aspartic acid.

8. Mutant hIL-4 proteins according to Claim 7, **characterized in that** tyrosine is replaced by glycine.

9. Process for preparing mutant hIL-4 proteins according to one of Claims 6 to 8, **characterized in that**
- cDNA, which encompasses a DNA region which encodes the mature region of hIL-4, is subjected to a targeted oligonucleotide mutagenesis (site-directed mutagenesis) such that a selected different amino acid of the possible natural amino acids, is expressed at the desired position(s),
- the DNA region which encodes the mutated, mature region of hIL-4 is inserted into an expression vector,
- the hybrid vector which has been formed is introduced into E. coli, yeast, or another eukaryotic cell, and
- the mutant hIL-4 proteins are expressed and, where appropriate, isolated.

10. Process according to Claim 9, **characterized in that** DNA encoding an initiating methionine is incorporated into the cDNA which encompasses the DNA region which encodes the mature region of hIL-4 prior to the site-directed mutagenesis being carried out.

11. Process according to Claim 9 or 10, **characterized in that** the DNA region which encodes the mutated, mature region of hIL-4 is excised from the vector as a NcoI/BamHI fragment.

12. Process according to one of Claims 9 to 11, **characterized in that** use is made of a temperature-regulated expression vector, for example pILA502 (without left-hand lambda promoter and polylinker) and/or of the modified E. coli strain JM103 (recA) as the host.

## Revendications

1. Utilisation de protéines mutantes de l'Il-4 humaine, qui sont des antagonistes ou des agonistes partiels de l'IL-4 humaine, où l'acide aminé ou les acides aminés apparaissant naturellement dans le type sauvage en une ou plusieurs des positions 121, 124 ou 125 est ou sont échangés contre un ou plusieurs des autres acides aminés naturels possibles, pour la préparation de médicaments.

2. Utilisation suivant la revendication 1, **caractérisée en ce que** la tyrosine apparaissant naturellement comme acide aminé en position 124 dans les protéines mutantes de l'IL-4 humaine, y est échangée contre un autre des acides aminés naturels possibles.

3. Utilisation suivant la revendication 2, **caractérisée en ce que** la tyrosine en position 124 est échangée contre l'acide aspartique ou contre la glycine.

4. Utilisation suivant la revendication 1, **caractérisée en ce que** l'arginine apparaissant naturellement comme acide aminé en position 121 dans les protéines mutantes de l'IL-4 humaine, y est échangée contre un autre des acides aminés naturels possibles.

5. Utilisation suivant la revendication 4, **caractérisée en ce que** l'arginine en position 121 est échangée contre l'acide aspartique ou contre la glycine.

6. Protéines mutantes de l'IL-4 humaine, **caractérisées en ce que** l'acide aminé ou les acides aminés apparaissant naturellement en une ou plusieurs des positions 121 ou 125 ainsi que, facultativement, en position 124 y est ou y sont échangés contre un autre des acides aminés naturels possibles.

7. Protéines mutantes de l'IL-4 humaine, **caractérisées en ce que** la tyrosine apparaissant naturellement comme acide aminé en position 124 y est échangée contre l'un des autres acides aminés naturels possibles, à l'exception de l'acide aspartique.

8. Protéines mutantes de l'IL-4 humaine suivant la revendication 7, **caractérisées en ce que** la tyrosine est échangée contre la glycine.

9. Procédé de préparation de protéines mutantes de l'IL-4 humaine suivant l'une des revendications 6 à 8, **caractérisé en ce que** :
- on soumet un ADNc comprenant une région d'ADN qui code la région mature de l'IL-4 humaine à une mutagenèse oligonucléotidique dirigée (site-directed-mutagenisis) de telle sorte qu'un acide aminé choisi parmi les autres acides aminés naturels possibles soit exprimé dans la ou les positions souhaitées,
- on insère dans un vecteur d'expression la région d'ADN qui code la région mature mutée de l'IL-4 humaine,
- on introduit le vecteur hybride formé dans E. coli, une levure ou une autre cellule d'eucaryote et
- on isole le cas échéant les protéines mutantes de l'IL-4 qui sont alors exprimées.

10. Procédé suivant la revendication 9, **caractérisé en ce qu'**on incorpore à l'ADNc comprenant la région d'ADN qui code la région mature de l'IL-4 humaine, l'ADN pour une méthionine initiale avant d'effectuer la mutagenèse oligonucléotidique dirigée.

11. Procédé suivant la revendication 9 ou 10, **caractérisé en ce qu'**on sépare par coupure du vecteur, comme fragment Ncol/BamHI, la région d'ADN qui code la région mature mutée de l'IL-4 humaine.

12. Procédé suivant l'une des revendications 9 à 11, **caractérisé en ce qu'**on utilise comme hôte un vecteur d'expression à température réglée, par exemple PILA 502 (sans promoteur lambda du côté gauche ni polylinker) et/ou la souche JM103 modifiée (recA) de E. coli.
